# EUROPEAN PATENT APPLICATION

(11) **EP 4 417 186 A1**
(43) Date of publication of application: **21.08.2024**
(21) Application number: 22880886.1
(22) Date of filing: 05.10.2022
(51) Int. Cl.: A61K 8/37, A61K 8/02, A61Q 17/04, C09K 3/00

(54) **ULTRAVIOLET ABSORBING POWDER, COSMETIC CONTAINING ULTRAVIOLET ABSORBING POWDER, DISPERSION, MIXED POWDER, AND METHOD FOR MANUFACTURING ULTRAVIOLET ABSORBING POWDER**

(30) Priority: 13.10.2021 JP 2021168432
(71) Applicant: Shiseido Company, Ltd., Chuo-ku Tokyo 104-0061 (JP)
(72) Inventor: NASU, Akio, Tokyo 104-0061 (JP); ITO, Ryoya, Tokyo 104-0061 (JP); KANG, Jasmin, Tokyo 104-0061 (JP)
(74) Representative: Cabinet Beau de Loménie
(86) International application number: PCT/JP2022/037291
(87) International publication number: WO 2023/063190

(57) **Abstract**

Provided is an ultraviolet absorbing powder in which the ultraviolet absorption ability in the UVA and UVB regions is further improved, a cosmetic including an ultraviolet absorbing powder, and a production method for this ultraviolet absorbing powder.

The cosmetic includes an ultraviolet absorbing powder, wherein the ultraviolet absorbing powder is composed of particles of a compound I having a structure of formula (I) below, and in the ultraviolet absorbing powder, particles of the compound I having a longest diameter exceeding 500 µm are 1.0 or less per 100 particles of the compound I. 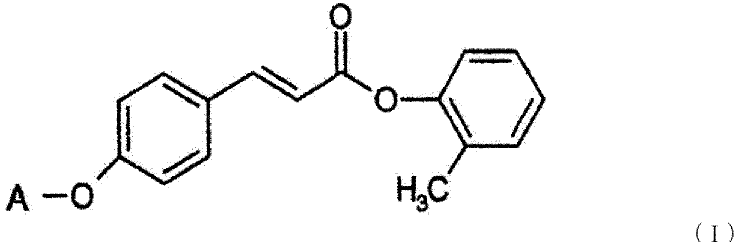 (where -OA represents an alkoxy group)

## Description

### FIELD

The present invention relates to an ultraviolet absorbing powder, a cosmetic, a dispersion, and a mixed powder comprising an ultraviolet absorbing powder, and a production method for an ultraviolet absorbing powder.

### BACKGROUND

Protecting the skin from ultraviolet rays is an important issue in skin care and body care. In addition to medium-wavelength ultraviolet rays (UVB region: wavelength 290 to 320 nm), which are known to cause sunburn and inflammation, long-wavelength ultraviolet rays (UVA region: wavelength 320 to 400 nm) are known to have an effect on the skin (photoaging, etc.).

Conventional cosmetics for ultraviolet protection often contain 2-ethylhexyl p-methoxycinnamate as an ultraviolet absorber. Though 2-ethylhexyl p-methoxycinnamate has the effect of absorbing ultraviolet rays in the UVB region, there is a need for an ultraviolet absorber having better ultraviolet absorption ability in the UVA region.

In connection thereto, the ultraviolet absorber disclosed in Patent Literature 1 has absorption ability in both the UVA region and the UVB region, has an ultraviolet suppression effect in a wide range of wavelengths, has a previously unseen property: the ultraviolet absorption capability thereof increases in the UVA and UVB regions with the passage of time of ultraviolet irradiation, and further, has excellent solubility.

More specifically, the ultraviolet absorber of Patent Literature 1 contains, as an active ingredient, a compound represented by general formula I:
[Chem 1] 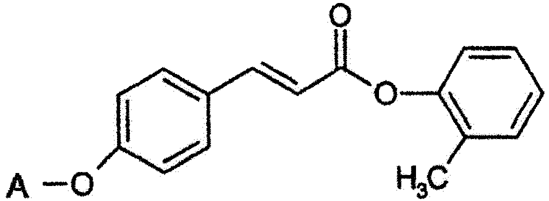 where -OA represents an alkoxy group.

### [CITATION LIST]

### [PATENT LITERATURE]

[PTL 1] WO 2009/041098

### SUMMARY

### [TECHNICAL PROBLEM]

In Patent Literature 1, the compound represented by general formula I above is blended into an ointment or into a liquid medium as-is, and exhibits ultraviolet absorption ability in the UVA and UVB regions.

However, the ultraviolet absorber of Patent Literature 1 still has room for improvement in terms of ultraviolet absorption ability in the UVA and UVB regions.

The preset invention aims to improve the situation described above, and an object thereof is to provide an ultraviolet absorbing powder having further improved ultraviolet absorption ability in the UVA and UVB regions, a cosmetic comprising the ultraviolet absorbing powder, and a production method for this ultraviolet absorbing powder.

### [SOLUTION TO PROBLEM]

The present invention, which can achieve the object described above, is as follows.

### <Aspect 1>

A cosmetic, comprising an ultraviolet absorbing powder,
wherein the ultraviolet absorbing powder is composed of particles of a compound I having a structure of formula (I) below: 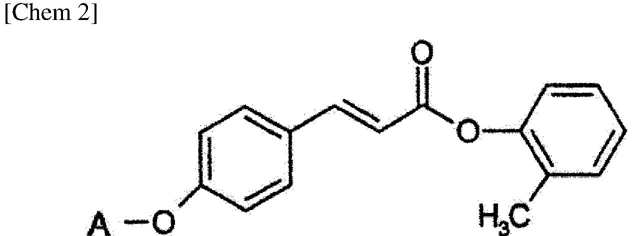
where -OA represents an alkoxy group, and(I)
wherein in the ultraviolet absorbing powder, particles of the compound I having a longest diameter exceeding 500 µm are 1.0 or less per 100 particles of the compound I.

### <Aspect 2>

The cosmetic according to Aspect 1, wherein an average particle diameter of the ultraviolet absorbing powder is 350 µm or less.

### <Aspect 3>

The cosmetic according to Aspect 1 or 2, comprising a liquid medium,
wherein the ultraviolet absorbing powder is dispersed or settled in the liquid medium.

### <Aspect 4>

The cosmetic according to Aspect 3, wherein the liquid medium is nonvolatile, and a solubility of the ultraviolet absorbing powder in the liquid medium is 40 mg/ml or less.

### <Aspect 5>

The cosmetic according to Aspect 1 or 2, which is a powder cosmetic.

### <Aspect 6>

The cosmetic according to Aspect 5, further comprising an additional powder other than the ultraviolet absorbing powder.

### <Aspect 7>

The cosmetic according to Aspect 6, wherein the additional powder contains an extender pigment.

### <Aspect 8>

The cosmetic according to any one of Aspects 1 to 7, which is a sunscreen cosmetic.

### <Aspect 9>

An ultraviolet absorbing powder composed of particles of a compound I having a structure of formula (I) below:
[Chem 3] 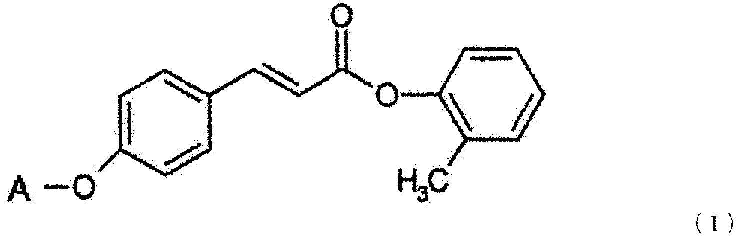
where -OA represents an alkoxy group, and
wherein particles of the compound I having a longest diameter exceeding 500 µm are 1.0 or less per 100 particles of the compound I.

### <Aspect 10>

A dispersion, comprising a liquid medium, and
wherein the powder according to Aspect 9 is dispersed or settled in the liquid medium.

### <Aspect 11>

A mixed powder, comprising:
the powder according to Aspect 9, and
an additional powder other than the powder according to Aspect 9.

### <Aspect 12>

The powder according to Aspect 11, wherein the additional powder contains an extender pigment.

### <Aspect 13>

A production method for an ultraviolet absorbing powder, comprising pulverizing particles of a compound I having a structure of formula (I) below:
[Chem 4] 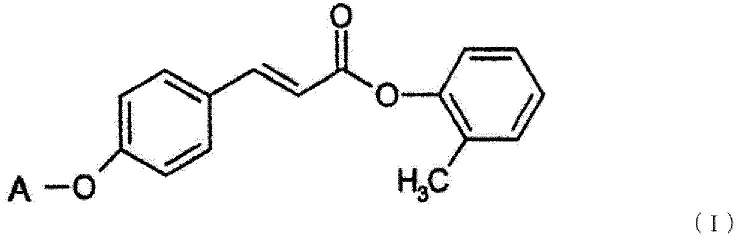 where -OA represents an alkoxy group.

### <Aspect 14>

The production method according to Aspect 13, wherein the pulverizing is such that particles of the compound I having a longest diameter exceeding 500 µm are 1.0 or less per 100 particles of the compound I.

### <Aspect 15>

The production method according to Aspect 13 or 14, wherein the pulverizing is performed using a wet pulverizer.

### <Aspect 16>

The production method according to Aspect 15, wherein a roller mill is used as the wet pulverizer.

### <Aspect 17>

The production method according to Aspect 15 or 16, wherein the pulverizing is performed after dispersing the particles of the compound I in a liquid medium.

### <Aspect 18>

The production method according to Aspect 13 or 14, wherein the pulverizing is performed using a dry pulverizer.

### <Aspect 19>

The production method according to Aspect 18, wherein the pulverizing is performed along with additional particles other than the particles of the compound I.

### <Aspect 20>

The production method according to Aspect 19, wherein the additional particles comprise an extender pigment.

### [ADVANTAGEOUS EFFECTS OF INVENTION]

According to the present invention, there can be provided an ultraviolet absorbing powder having further improved ultraviolet absorption ability in the UVA and UVB regions, a cosmetic comprising an ultraviolet absorbing powder, and a production method for this ultraviolet absorbing powder.

### BRIEF DESCRIPTION OF DRAWINGS

FIG. 1 is a microscopic observation image of the raw material powder of Comparative Example 1.
FIG. 2 is a microscopic observation image of the ultraviolet absorbing powder of Example 1.
FIG. 3 is a microscopic observation image of the ultraviolet absorbing powder of Example 2.
FIG. 4 is a microscopic observation image of the ultraviolet absorbing powder of Example 3.
FIG. 5 is a microscopic observation image of the ultraviolet absorbing powder of Example 4.
FIG. 6 is a microscopic observation image of the ultraviolet absorbing powder of Example 5.
FIG. 7 is a microscopic observation image of Example 6.
FIG. 8 is a view showing the ultraviolet absorption spectra of the ultraviolet absorbing powders of Examples 1 to 6 and Comparative Example 1.
FIG. 9 is a view showing the ultraviolet absorption spectra of the compositions of Examples 7 to 12 and Comparative Examples 2 and 3.

### DESCRIPTION OF EMBODIMENTS

The embodiments of the present invention will be described below. Note that the present invention is not limited to the following embodiments, and various changes can be made within the scope of the spirit of the invention.

### <<Cosmetic>>

One aspect of the present invention relates to a cosmetic.

The cosmetic of the present invention is:
a cosmetic, comprising an ultraviolet absorbing powder, wherein
the ultraviolet absorbing powder is composed of particles of a compound I having a structure of formula (I) below: 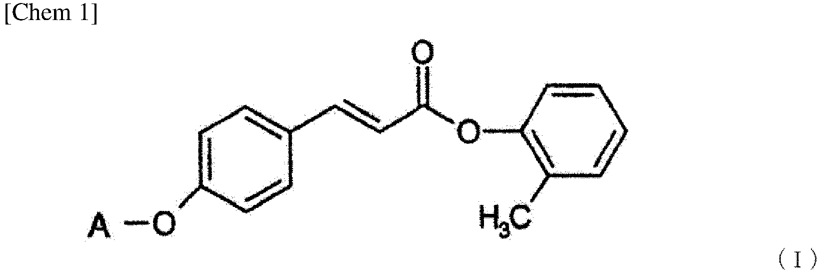
   where -OA represents an alkoxy group, and
   wherein in the ultraviolet absorbing powder, particles of the compound I having a longest diameter exceeding 500 µm are 1.0 or less per 100 particles of the compound I.

Since the cosmetic of the present invention has high ultraviolet absorption ability in the UVA and UVB regions by comprising a specific ultraviolet absorbing powder with a specific particle diameter distribution, it can suitably be used as a sunscreen cosmetic.

In formula (I), -OA represents an alkoxy group, and more specific examples thereof include, but are not limited to, a methoxy group and an ethoxy group.

The chemical structure of the compound I constituting the ultraviolet absorbing powder included in the cosmetic of the present invention (hereinafter also simply referred to as the "ultraviolet absorbing powder of the present invention") may be the same as the structure of the compound disclosed in Patent Literature 1.

Regarding such compound I, the present inventors have discovered that by:
(i) reducing the particle diameter of the powder of the compound I to a specific size, and
(ii) blending the powder of the compound I having a reduced particle diameter into a cosmetic in a powder state,
an extremely excellent ultraviolet absorption ability in the UVA and UVB regions can be provided.

Regarding the degree of smallness of the particle diameter of the powder of the compound I, particles of the compound I having a longest diameter exceeding 500 µm may be 1.0 or less per 100 particles of the compound I. Thus, the ultraviolet absorbing powder of the present invention can be defined as an aggregate in which particles of the compound I having a longest diameter exceeding 500 µm are 1.0 or less per 100 particles of the compound I.

Note that in the present invention, "longest diameter" means the longest diameter of a particle when observed with an optical microscope, and thus, for example, if a particle observed with an optical microscope is circular, the diameter corresponds to the longest diameter, and if a particle observed with the optical microscope is rectangular, the length of the diagonal corresponds to the longest diameter.

Furthermore, in the present invention, the method for measuring the "average particle diameter" regarding the ultraviolet absorbing powder of the present invention is not particularly limited, and for example, the following method (i) or (ii) may be used.

### Measurement Method (i)

The "average diameter particle" can be determined as a volume-based particle diameter, i.e., the volume average particle diameter (D50), as measured using a laser diffraction/scattering particle diameter distribution measuring device (MT3300EXII; manufactured by Microtrac Bell) by adding the powder to a suitable solvent and then performing ultrasonic dispersion for one minute using an ultrasonic homogenizer (US-150T; manufactured by NISSEI Corporation).

### Measurement Method (ii)

The "average diameter particle" can be determined by diluting the powder with an appropriate solvent, capturing a photograph using a microscope (BX51, manufactured by Olympus Corporation), measuring the diameter of approximately 100 randomly selected particles in the photograph obtained in this manner, and calculating the average value.

Furthermore, the "average particle diameter" of a powder other than the ultraviolet absorbing powder of the present invention, such as an ultraviolet scattering powder or extender pigment, may be determined as, for example, the average value of the circle equivalent diameter equivalent of the projected area of the primary particles in the SEM image.

From the viewpoint of exhibiting the effects of the present invention, it is preferable that the number of particles of the compound I having a longest diameter exceeding 500 µm be few. For example, in the ultraviolet absorbing powder of the present invention, the number of particles of the compound I having a longest diameter exceeding 500 µm may be, per 100 particles of the compound I, 1.0 or less, 0.9 or less, 0.8 or less, 0.7 or less, 0.6 or less, 0.5 or less, 0.4 or less, 0.3 or less, 0.2 or less, or 0.1 or less. Furthermore, the lower limit of the number of particles of the compound I having a longest diameter exceeding 500 µm is not particularly limited, and may be, per 100 particles of the compound I, 0.001 or more or 0.005 or more.

Furthermore, from the viewpoint of further exhibiting the effects of the present invention, in the ultraviolet absorbing powder of the present invention,
(i) the number of particles of the compound I having a longest diameter exceeding 400 µm may be 1.0 or less per 100 particles of the compound I,
(ii) the number of particles of the compound I having a longest diameter exceeding 300 µm may be 1.0 or less per 100 particles of the compound I,
(iii) the number of particles of the compound I having a longest diameter exceeding 200 µm may be 1.0 or less per 100 particles of the compound I, or
(iv) the number of particles of the compound I having a longest diameter exceeding 100 µm may be 1.0 or less per 100 particles of the compound I.

Furthermore, from the viewpoint of exhibiting the effects of the present invention, in any of the above cases (i) to (iv), the number of particles of the compound I having the longest diameter described above may be, per 100 particles of the compound I, 1.0 or less, 0.9 or less, 0.8 or less, 0.7 or less, 0.6 or less, 0.5 or less, 0.4 or less, 0.3 or less, 0.2 or less, or 0.1 or less. Furthermore, the lower limit of the number of particles of the compound I having the particle diameter described above is not particularly limited, and may be, for example, per 100 particles of the compound I, 0.001 or more or 0.005 or more.

The average particle diameter of the ultraviolet absorbing powder of the present invention may be 350 µm or less, 300 µm or less, 250 µm or less, 220 µm or less, 200 µm or less, 150 µm or less, or 100 µm or less, and may be 1.0 µm or more, 5.0 µm or more, or 10 µm or more.

Regarding the present invention, the phrase a cosmetic "comprising an ultraviolet absorbing powder" means that the cosmetic comprises the ultraviolet absorbing powder in powder form.

### <Cosmetic Form>

The form of the cosmetic of the present invention is not particularly limited, and the cosmetic of the present invention may be, for example, a cream cosmetic, a gel cosmetic, a liquid cosmetic, or a powder-based powder cosmetic. Examples of the cream cosmetic, gel cosmetic, and liquid cosmetic include, but are not limited to, oil-in-water emulsion cosmetics, water-in-oil emulsion cosmetics, and oil content-based oil cosmetics.

### (Ointment Cosmetic, Cream Cosmetic, Gel Cosmetic, Liquid Cosmetic)

In the case of an ointment cosmetic, a cream cosmetic, a gel cosmetic, or a liquid cosmetic, the cosmetic of the present invention contains an ointment or a medium, in particular, a cream, gel or liquid medium, and the ultraviolet absorbing powder may be dispersed or settled in the medium. In the present invention, the term "medium" refers to an ointment, cream, gel, liquid, etc., in which the ultraviolet absorbing powder is dispersed or settled. Furthermore, "ointment" refers to an oil-based product, and "cream" refers to an emulsion-based product of a mixture of water and oil.

When the cosmetic of the present invention is an ointment cosmetic, a cream cosmetic, a gel cosmetic, or a liquid cosmetic, the content of the medium is not particularly limited, and may be, for example, 10 to 99% by mass relative to the total amount of the cosmetic.

In the cosmetic of the present invention, when the ultraviolet absorbing powder of the present invention is dispersed or settled in a medium, it is preferable that the solubility of the ultraviolet absorbing powder of the present invention in such a medium be low. Thus, the solubility of the ultraviolet absorbing powder of the present invention in such a medium may be 40 mg/ml or less, and more specifically, may be, for example, 30 mg/ml or less, 20 mg/ml or less, 10 mg/ml or less, 5.0 mg/ml or less, 1.0 mg/ml or less, 0.5 mg/ml or less, 0.1 mg/ml or less, or approximately 0 mg/ml.

Note that this medium is preferably nonvolatile in order to stabilize the cosmetic composition.

In one aspect, the cosmetic of the present invention contains a liquid medium, and the ultraviolet absorbing powder is dispersed or settled in the liquid medium, and in particular, the liquid medium is nonvolatile, and the solubility of the ultraviolet absorbing powder in the liquid medium is 40 mg/ml or less.

When the cosmetic of the present invention is a liquid cosmetic and the ultraviolet absorbing powder is settled in the liquid medium, the cosmetic of the present invention may be a shaken cosmetic in which the cosmetic is shaken to disperse the ultraviolet absorbing powder in the liquid medium prior to use.

In the case of an ointment cosmetic, a cream cosmetic, a gel cosmetic, or a liquid cosmetic, the cosmetic of the present invention can be produced by, for example, mixing the components including the medium described above, and dispersing the ultraviolet absorbing agent of the present invention therein. Furthermore, in a state prior to production, i.e., in a state prior to pulverizing the powder of the compound I, the ultraviolet absorbing powder of the present invention may be mixed with an additional powder, they may be pulverized together, and the pulverized powder may then be mixed with the components including the medium and dispersed.

Examples of dispersion devices include media dispersion devices such as bead mills, sand mills, ball mills, and paint shakers, high-speed stirrers such as homogenizers and dispersers, and media-less dispersion devices such as high-pressure dispersion machines and ultrasonic dispersion machines. There are both horizontal and vertical bead mills, and either type may be used. Generally, the lower the temperature of a dispersion at the time of dispersion, the better the storage stability of a dispersion composition can be obtained. For example, the dispersing may be performed such that the temperature of a dispersion is 10 °C to 40 °C.

### (Medium)

When the cosmetic of the present invention is an ointment cosmetic, a cream cosmetic, a gel cosmetic, or a liquid cosmetic, the medium, and in particular, the cream, gel, or liquid medium, may be an aqueous medium or an oil-based medium. These media may be volatile or nonvolatile.

Specific examples of the medium include, but are not limited to, low polarity oil contents for example, hydrocarbon oils such as liquid paraffin, tetraisobutane, hydrogenated polydecene, olefin oligomers, isododecane, isohexadecane, squalane, and hydrogenated polyisobutene, and silicone oils such as cyclopentasiloxane, trisiloxane, caprylyl methicone, dimethicone 0.5 cst to 200 cst (silicone KF-96A-6T, etc.), amino-modified polysiloxane, polyether-modified polysiloxane, alkyl-modified polysiloxane, phenyl-modified siloxane, and fluorine-modified polysiloxane.

Furthermore, the polarity of the medium is not limited as long as the ultraviolet absorbing powder of the present invention is not easily dissolved therein. The medium may be a polar oil which is used as an ultraviolet absorber in cosmetics, and it may be, for example, octocrylene, bisethylhexyloxyphenol methoxyphenyl triazine, diethylaminohydroxybenzoylhexyl benzoate, ethylhexyl triazone, ethylhexyl methoxycinnamate, or 2-ethylhexyl 4-methoxycinnamate.

The medium may also be an oil content which is used as a nonvolatile or volatile oil in cosmetics.

In the present invention, "nonvolatile oil content" refers to oil content having a boiling point of 260 °C or higher under normal pressure. Examples of the nonvolatile oil content include, but are not limited to, nonvolatile dimethicone, caprylyl methicone, liquid paraffin, squalane, castor oil, olive oil, jojoba oil, glyceryl diisostearate, trimethylolpropane tri2-ethyl isostearate, isopropyl myristate, cetyl 2-ethylhexanoate, glyceryl triisostearate, 2-heptyl undecyl palmitate, methylpolysiloxane (degree of polymerization 4 or higher), polybutene, glyceryl triisostearate, diisostearyl malate, and lanolin. Two or more of these nonvolatile oil contents may be arbitrarily selected and combined.

In the present invention, "volatile oil content" means an oil content having a boiling point of lower than 260 °C under normal pressure. Examples of the volatile oil content which can be used in the present invention include chain polysiloxanes (degree of polymerization 4 or more) such as decamethyltetrasiloxane, hexamethyldisiloxane, and dodecamethylpentasiloxane, cyclic polysiloxanes (4 to 6 silicon atoms) such as octamethylcyclotetrasiloxane and decamethylcyclopentasiloxane, and light liquid isoparaffins such as ShellSol (Shell Chemical) and Isopar (Esso Chemical). Two or more of these volatile oil contents may be arbitrarily selected and used in combination.

### (Powder Components)

In the cosmetic of the present invention, examples of powders that can be used in addition to the ultraviolet absorber powder of the present invention include, but are not limited to, ultraviolet scattering powders and usability powders.

### (Powder Component - Ultraviolet Scattering Powder)

The ultraviolet scattering powder is not particularly limited, and may be a powder of, for example, at least one selected from the group consisting of titanium oxide, zinc oxide, iron oxide, and cerium oxide.

Furthermore, the ultraviolet scattering powder may be subjected to a hydrophobization treatment. The hydrophobization treatment may be performed using a known surface treatment agent for hydrophobization, such as a fluorine compound treatment, silicone treatment, silicone resin treatment, pendant treatment, silane coupling agent treatment, titanium coupling agent treatment, oil treatment, N-acylated lysine treatment, polyacrylic acid treatment, metal soap treatment, amino acid treatment, inorganic compound treatment, plasma treatment, mechanochemical treatment, silane compound treatment, and silazane compound treatment. Among these treatments, from the viewpoint of dispersion stability, etc., a treatment with silicone or a silicone resin, treatment with a silane compound or silazane compound, and treatment with a metal soap such as aluminum isostearate are preferable.

The average particle diameter of the ultraviolet scattering powder is not particularly limited, and may be, for example, 5 nm or more, 10 nm or more, or 15 nm or more, and may be 200 nm or less, 100 nm or less, or 50 nm or less.

When the cosmetic of the present invention contains an ultraviolet scattering powder, the content thereof is not particularly limited, and may be, for example, relative to the total amount of the cosmetic, 0.1% by mass or more, 0.5% by mass, 1.0% by mass or more, or 5.0% by mass or more, and may be 35% by mass or less, 30% by mass or less, 25% by mass or less, 20% by mass or less, 10% by mass, or 8.0% by mass or less.

### (Powder Component- Usability Powder)

In the present invention, "usability powder" refers to any powder that has the function of adjusting the usability of a cosmetic, such as the feel (for example, dry feel, smooth feel, or moist feel).

In the present invention, the usability powder is not particularly limited, and may be, for example, at least one powder selected from the group consisting of an extender pigment, resin powder, silica powder, cellulose powder, and starch powder. Further, these powders may be subjected to a hydrophobization treatment.

In the present invention, an extender pigment is an inorganic pigment which is generally used to maintain the dosage form of a cosmetic, and by incorporating an extender pigment, the usability (spreadability, adhesion), gloss, color tone, etc., of the cosmetic can be adjusted. Further, when the cosmetic of the present invention is a powder cosmetic, by including an extender pigment, the storage stability of the cosmetic can be improved. Furthermore, the extender pigment can also be pulverized along with the compound I described above.

Specific examples of the extender pigment include, but are not limited to, barium sulfate powders, boron nitride powders, talc, kaolin, mica, synthetic mica, synthetic phlogopite iron, sericite, magnesium carbonate, calcium carbonate, talc/silica/titanium oxide composites, and fatty acid magnesium. Furthermore, the cosmetic of the present invention may contain a combination of two or more extender pigments. Among these, it is preferable to use one or more selected from talc, kaolin, mica, and synthetic phlogopite iron.

The average particle diameter of the extender pigment is not particularly limited, and may be, for example, 5 nm or more, 10 nm or more, or 15 nm or more, and may be 200 nm or less, 100 nm or less, or 50 nm or less.

When the cosmetic of the present invention contains an extender pigment, the content thereof is not particularly limited, and may be, relative to the total amount of the cosmetic, for example, 1.0% by mass or more, 2.0% by mass or more, 5.0% by mass or more, or 10% by mass or more, and may be 80% by mass or less, 70% by mass or less, 60% by mass or less, 50% by mass or less, 40% by mass or less, 30% by mass or less, 20% by mass or less, or 10% by mass or less.

In the present invention, the resin powder is not particularly limited, and examples thereof include, but are not limited to, urethane resin powders, nylon powders, and crosslinked acrylic resin (PMMA resin) powders. Further, the resin powder may be spherical.

In the invention, the silica powder is not particularly limited, and examples thereof include, but are not limited to, powders of silica itself, hydrous silica powders, and silica silicone rubber powders. Further, the silica powder may be spherical.

In the present invention, when a usability powder is included, the content thereof is not particularly limited, and it may be, relative to the total amount of the cosmetic, for example, 1.0% by mass or more, 5.0% by mass or more, 8.0% by mass or more, 10% by mass or more, 20% by mass or more, or 30% by mass or more, and may be 80% by mass or less, 70% by mass or less, 60% by mass or less, 50% by mass or less, 40% by mass or less, 35% by mass or less, or 30% by mass or less.

### (Additional Additive Components)

The cosmetic of the present invention may further include additional components such as surfactants, moisturizers, higher alcohols, sequestering agents, natural and synthetic polymers, water-soluble and oil-soluble polymers, various extracts, colorants such as organic dyes, preservatives, antioxidants, pigments, thickeners, chelating agents, pH adjusters, fragrances, cooling agents, antiperspirants, disinfectants, skin activators, other drugs, and various powders, but the additional components are not limited thereto.

Further, the cosmetic of the present invention may further contain, in addition to the above-mentioned components, any aqueous components which can be used in the field of cosmetics.

### (Powder Cosmetic)

The cosmetic of the present invention may be a powder cosmetic.

In the case of a powder cosmetic, the cosmetic of the present invention may contain only the ultraviolet absorbing powder of the present invention, and optionally may further contain an additional powder other than the ultraviolet absorbing powder of the present invention, or optionally may further contain additive components such as various oil contents (volatile oil contents or nonvolatile contents), coloring agents, or stabilizers.

In the case of a powder cosmetic, the cosmetic of the present invention may be produced by, for example, pulverizing the particles of the compound I to obtain an ultraviolet absorbing powder, then mixing with an additional powder the ultraviolet absorbing powder of the present invention obtained by the pulverizing, or may be produced by pulverizing the particles of the compound I along with additional particles.

In the case of a powder cosmetic, the cosmetic of the present invention can contain additional components in addition to the ultraviolet absorbing powder of the present invention. Examples of these components include the components described above as the medium, powder component, and additional additive components.

The medium, powder component, and additional additive components can be used in the amounts described above within appropriate ranges for a powder cosmetic. In particular, when the cosmetic of the present invention contains a component described above as the medium, and in particular, a component described above as an oil content, the content thereof is not particularly limited, and may be, relative to the total amount of the cosmetic, for example, 1.0% by mass or more, 2.0% by mass or more, 3.0% by mass or more, 4.0% by mass or more, or 5.0% by mass or more, and may be 20% by mass or less or 10% by mass or less.

### <<Ultraviolet Absorbing Powder, Dispersion, and Mixed Powder>>

In one aspect, the present invention relates to an ultraviolet absorbing powder, a dispersion, and a mixed powder.

The ultraviolet absorbing powder of the present invention is an ultraviolet absorbing powder composed of particles of the compound I described above, in which particles of the compound I having a longest diameter exceeding 500 µm are 1.0 or less per 100 particles of the compound I.

The dispersion of the present invention comprises a liquid medium, and the powder of the present invention is dispersed or settled in the liquid medium. Furthermore, the mixed powder of the present invention comprises the powder of the present invention and an additional powder other than the powder of the present invention, such as an extender pigment.

Regarding the details of the ultraviolet absorbing powder, dispersion, and mixed powder of the present invention, refer to the descriptions above regarding the cosmetic of the present invention.

### < <Production Method for Ultraviolet Absorbing Powder>>

In one aspect, the present invention relates to a production method for an ultraviolet absorbing powder.

The production method for an ultraviolet absorbing powder of the present invention comprises pulverizing particles of the compound I described above.

In this production method, the pulverizing may be such that particles of the compound I having a longest diameter exceeding 500 µm are 1.0 or less per 100 particles of the compound I.

From the viewpoint of exhibiting the effects of the present invention, it is preferable that the number of particles of the compound I having a longest diameter exceeding 500 µm be few. For example, in the ultraviolet absorbing powder obtained by this production method, the number of particles of the compound I having a longest diameter exceeding 500 µm, per 100 particles of the compound I, may be 1.0 or less, 0.9 or less, 0.8 or less, 0.7 or less, 0.6 or less, 0.5 or less, 0.4 or less, 0.3 or less, 0.2 or less, or 0.1 or less. Furthermore, the lower limit of the number of particles of the compound I having a longest diameter exceeding 500 µm is not particularly limited, and may be, per 100 particles of the compound I, 0.001 or more or 0.005 or more.

Furthermore, from the viewpoint of further exhibiting the effects of the present invention, in the ultraviolet absorbing powder obtained by this production method,
(i) the number of particles of the compound I having a longest diameter exceeding 400 µm may be 1.0 or less per 100 particles of the compound I,
(ii) the number of particles of the compound I having a longest diameter exceeding 300 µm may be 1.0 or less per 100 particles of the compound I,
(iii) the number of particles of the compound I having a longest diameter exceeding 200 µm may be 1.0 or less per 100 particles of the compound I, or
(iv) the number of particles of the compound I having a longest diameter exceeding 100 µm may be 1.0 or less per 100 particles of the compound I.

Further, from the viewpoint of exhibiting the effects of the present invention, in any of the above cases (i) to (iv), the number of particles of the compound I having a longest diameter as described above, per 100 particles of the compound I, may be 1.0 or less, 0.9 or less, 0.8 or less, 0.7 or less, 0.6 or less, 0.5 or less, 0.4 or less, 0.3 or less, 0.2 or less, or 0.1 or less. Furthermore, the lower limit of the number of particles of the compound I having a longest diameter as described above is not particularly limited, and may be, per 100 particles of the compound I, for example, 0.001 or more or 0.005 or more.

The average particle diameter of the ultraviolet absorbing powder obtained by this production method may be 350 µm or less, 300 µm or less, 250 µm or less, 220 µm or less, 200 µm or less, 150 µm or less, or 100 µm or less, and may be 1.0 µm or more, 5.0 µm or more, or 10 µm or more.

Furthermore, the pulverizing may be wet pulverization, dry pulverization, or a combination of wet pulverization and dry pulverization.

In the case of wet pulverization, the pulverizing may be performed using a wet pulverizer. As the wet pulverizer, for example, a colorant dispersion device such as a roller mill may be used. The roller mill may comprise, for example, three rollers: a feed roller, an intermediate roller, and a finishing roller, which rotate at a low speed, a medium speed, and a high speed, respectively. The rotational speeds of the rollers are not particularly limited, and may be appropriately set in accordance with the amount of raw material of the ultraviolet absorbing powder (for example, particles of the compound I, etc.) used, the required diameter of the particles of the ultraviolet powder, etc.

In the case of wet pulverization, the pulverizing may be performed after the particles of the compound I described above are dispersed in a liquid medium. In this case, the amount of particles of the compound I dispersed in the liquid medium is not particularly limited, and may be, relative to the total amount of the liquid medium and the particles of the compound I, for example, 10% by mass or more, 15% by mass or more, or 20% by mass or more, and may be 50% by mass or less, 40% by mass or less, 30% by mass or less, or 20% by mass or less.

In the case of dry pulverization, the pulverizing may be performed using a dry pulverizer. As the dry pulverizer, an Agi-Homo Mixer, an atomizer, or the like may be used, and from the viewpoint of increasing pulverizing efficiency, it is preferable that an atomizer be used.

An Agi-Homo Mixer referred to also as a "vacuum emulsification device" may comprise a stirring tank, a homomixer, an inner surface scraping mechanism, and a heating/cooling device. When pulverizing, the turbine blades of the stator are rotated at high speed, whereby the raw material of ultraviolet absorbing powder is constantly suctioned from the bottom, in the manner of a pump, and circulated and stirred inside the tank.

An atomizer referred to also as a "powder pulverizing device" has a pulverizing chamber, and the pulverizing chamber may comprise therein, for example, a lining plate, a screen, a hammer, or a hammer plate. When pulverizing, the raw material of ultraviolet absorbing powder is supplied to the pulverizing chamber by, for example, a hopper, and through the action of a hammer rotating at high speed, is pulverized due to collision with the lining plate, mutual collision, and friction.

In the case of dry pulverization, particles of the compound I may be pulverized along with additional particles other than particles of the compound I. The additional particles are not particularly limited, and from the viewpoint of improving usability and storage stability, it is preferable that an extender pigment be included. The types of the extender pigment are as described above.

The shape of the additional particles added in addition to particles of the compound I when pulverizing is not particularly limited, and may be, for example, plate-like or scale-like. Further, the amount of additional particles to be added is not particularly limited, and for example, the ratio of the weight of the additional particles to the weight of the particles of the compound I (additional particles/particles of the compound I) may be 0.1 or more, 0.2 or more, 0.5 or more, or 0.8 or more, and may be 10.0 or less, 8.0 or less, 5.0 or less, 3.0 or less, 2.0 or less, 1.5 or less, or 1.2 or less. This ratio may be, for example, 1.0.

The pulverization time is not particularly limited, and can be appropriately set in accordance with the type of pulverizing used (for example, wet pulverizing or dry pulverizing), the pulverizing device used, the amount of raw material of the ultraviolet absorbing powder used, etc. For example, when performing dry pulverization using an Agi-Homo Mixer, from the viewpoint of making the size of the particles of the compound I small and uniform, the pulverization time may be, for example, 1 minute or longer, 5 minutes or longer, 10 minutes or longer, 20 minutes or longer, 30 minutes or longer, 45 minutes or longer, or 60 minutes or longer, and from the viewpoint of maintaining efficiency and stability of the obtained particles, the pulverization time may be, for example, 60 minutes or less, 45 minutes or less, or 30 minutes or less.

### EXAMPLES

The present invention will be described in more detail with reference to Examples below, but the present invention is not limited thereto.

### <<Examples 1 to 6>>

In Examples 1 to 6, the raw material powder of compound 1 represented by the following structural formula (hereinafter simply referred to as "raw material powder") was pulverized, and in the obtained ultraviolet absorbing powder of each Example, particles having a longest diameter exceeding 500 µm was set so as to be 1.0 or less per 100 particles.

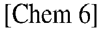

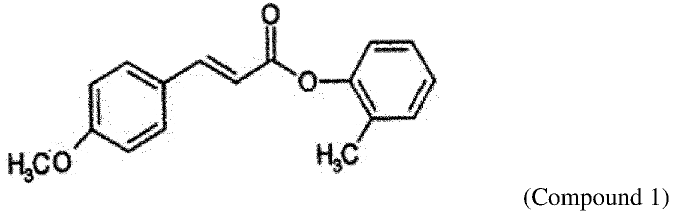

Note that in Examples 1 to 4, pulverizing was performed for 1 minute (Example 1), 10 minutes (Example 2), 30 minutes (Example 3), and 60 minutes (Example 4) in an Agi-Homo Mixer. In Example 5, pulverizing was performed using an atomizer. In Example 6, particles of the raw material compound were dispersed in nonvolatile dimethicone such that the particles were 40% by mass, and pulverizing was performed with a roller mill.

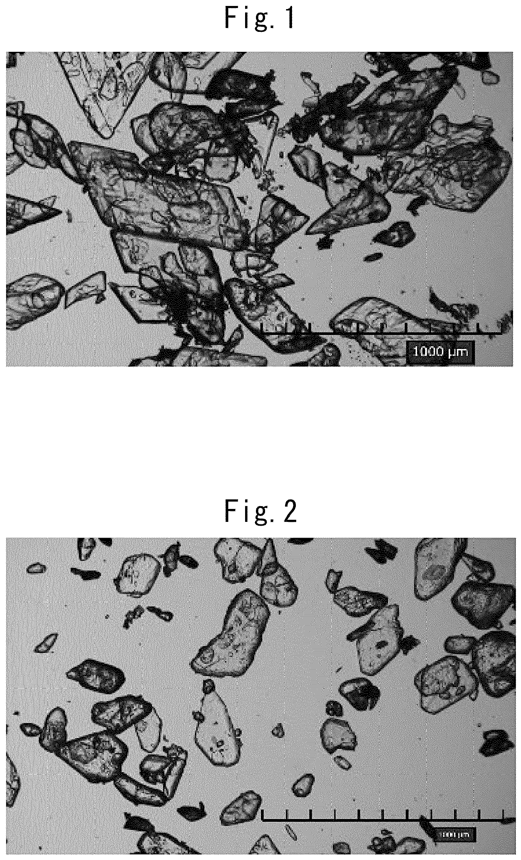

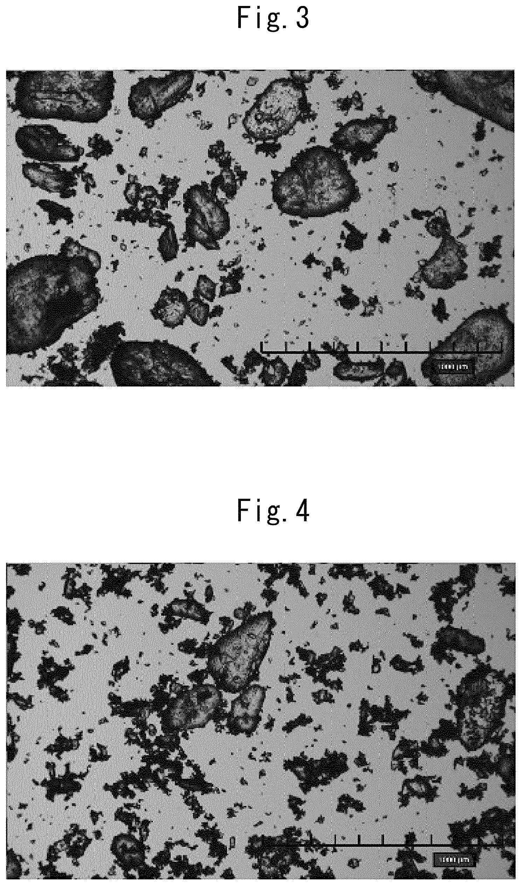

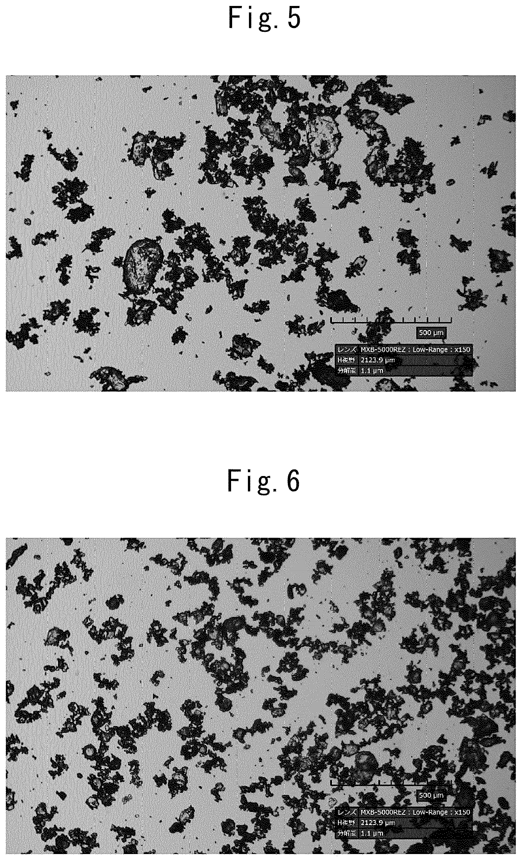

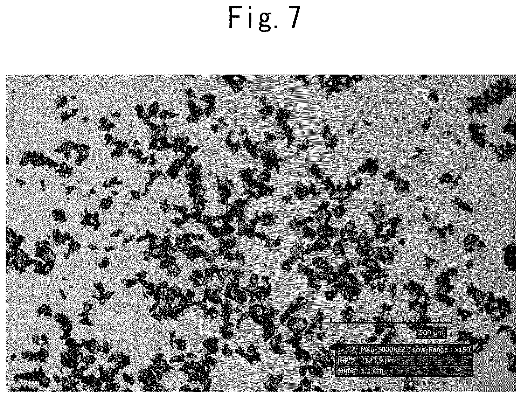

The obtained ultraviolet absorbing powders of Examples 1 to 5 were dispersed in nonvolatile dimethicone such that the ultraviolet absorbing powder was 40% by mass. Since Example 6 was obtained as an ultraviolet absorbing powder dispersed in nonvolatile dimethicone, it was used as-is as a dispersion liquid. The dispersion liquid of each Example was observed with an optical microscope. The respective results are shown in FIGS. 2 to 7 (FIG. 2: Example 1; FIG. 3: Example 2; FIG. 4: Example 3; FIG. 5: Example 4; FIG. 6: Example 5; and FIG. 7: Example 6) and shown in Table 1. Table 1 also shows the longest diameter of the largest particles observed in each of the Examples and Comparative Examples.

### < Comparative Example 1>>

The raw material powder was directly dispersed in nonvolatile dimethicone without being pulverized such that the raw material powder was 40% by mass. The obtained dispersion liquid was observed under a microscope in the same manner as in the above Examples, and the results are shown in FIG. 1 and Table 1.

[Table 1]

**(Table 1: Observation results of UV absorbing powders of Example 1 to 6 and raw material powder of Comparative Example 1)**

| | Example 1 | Example 2 | Example 3 | Example 4 | Example 5 | Example 6 | Comparative Example 1 |
|---|---|---|---|---|---|---|---|
| Whether there are 1.0 or more particles having longest diameter exceeding 500 µm per 100 particles | Absent | Absent | Absent | Absent | Absent | Absent | Present |
| Whether there are 1.0 or more particles having longest diameter exceeding 400 µm per 100 particles | Absent | Absent | Absent | Absent | Absent | Absent | Present |
| Whether there are 1.0 or more particles having longest diameter exceeding 300 µm per 100 particles | Present | Absent | Absent | Absent | Absent | Absent | Present |
| Whether there are 1.0 or more particles having longest diameter exceeding 200 µm per 100 particles | Present | Absent | Absent | Absent | Absent | Absent | Present |
| Longest diameter of largest particles (µm) (number of fine particles (50 µm or less)) | Approx. 400 (few fine particles) | Approx. 600 (many fine particles) | Approx. 350 (many fine particles) | Approx. 200 (many fine particles) | Approx. 100 (many fine particles) | Approx. 100 (many fine particles) | Approx. 750 (few fine particles) |

### <<Evaluation of Ultraviolet Absorption Ability>>

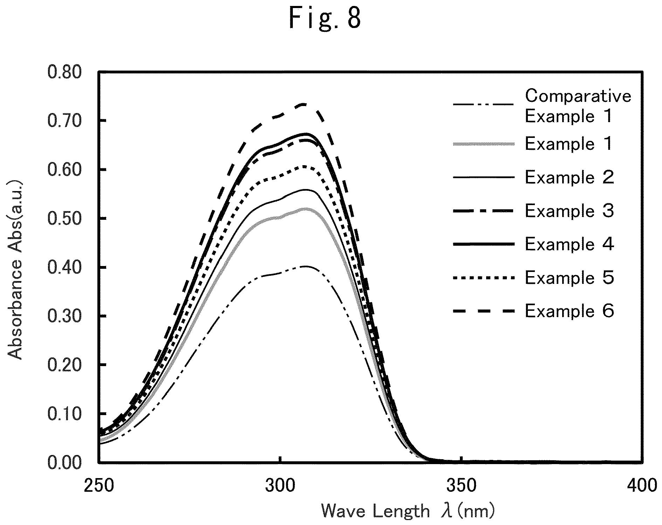

The nonvolatile dimethicone dispersions containing 40% by mass of the ultraviolet absorbing powders of Examples 1 to 6 and Comparative Example 1 were diluted 5000 times, placed in a 1 cm glass cell, and ultraviolet absorption spectra were measured. The results are shown in FIG. 8.

As is clear from the results in FIG. 8, as compared to Comparative Example 1, Examples 1 to 6 had significantly improved ultraviolet absorption ability in the UVA region (wavelength 320 to 400 nm) and UVB region (wavelength 290 to 320 nm).

### <<Examples 7 to 12 and Comparative Examples 2 and 3>>

Based on the compositions and production methods shown in Table 2 below, dispersions (Examples 8, 9, 11 and 12) and mixed powders (Examples 7 and 10) containing the ultraviolet absorbing powders of Examples 7 to 12 were prepared.

Note that in Examples 9 and 12, a combination of dry pulverization and wet pulverization was performed, and in both Examples, dry pulverization was performed first and then wet pulverization was performed.

In order to determine the average particle diameter of the obtained powder, a part of the obtained ultraviolet absorbing powder was diluted in an appropriate solvent (the solvent is not particularly limited and is preferably one that does not dissolve the ultraviolet absorbing powder). A photograph was then captured using a microscope (BX51, manufactured by Olympus Corporation), and the particle diameter of a part of the particles arbitrarily selected in the photograph obtained in such a manner was measured. The obtained particle diameter distribution is shown in Table 2.

[Table 2]

**(Table 2)**

| | | Example 7 | Example 8 | Example 9 | Example 10 | Example 11 | Example 12 |
|---|---|---|---|---|---|---|---|
| UV absorbing powder raw material | Compound 1 | 5.0 parts by mass | | | | | |
| Liquid medium | Silicone KF56 | - | 2.5 | 2.5 | - | 5.0 | 5.0 |
| | Silicone KF-96A-6T | - | 2.5 | 2.5 | - | 5.0 | 5.0 |
| Additional powder | Plate-like barium sulfate (extender pigment) | - | - | - | 5.0 | 5.0 | 5.0 |
| Total (parts by mass) | | 5.0 | 10 | 10 | 10 | 20 | 20 |
| Production method | Pulverization method | Dry | Wet | Dry and wet | Drv | Wet | Dry and wet |
| | Pulverizing device | Atomizer | Roller mill | Atomizer and roller mill | Atomizer | Roller mill | Atomizer and roller mill |
| Product | Whether there are 1.0 or more particles having longest diameter exceeding 500 µm per 100 particles | Absent | Absent | Absent | Absent | Absent | Absent |
| | Form | Mixed powder 1 | Dispersion (slurry) 1 | Dispersion (slurry) 2 | Mixed powder 2 | Dispersion (slurry) 3 | Dispersion (slurry) 4 |
| | Particle diameter distribution (µm) | 75 to 120 | 60 to 100 | 50 to 100 | 80 to 150 | 60 to 100 | 50 to 80 |

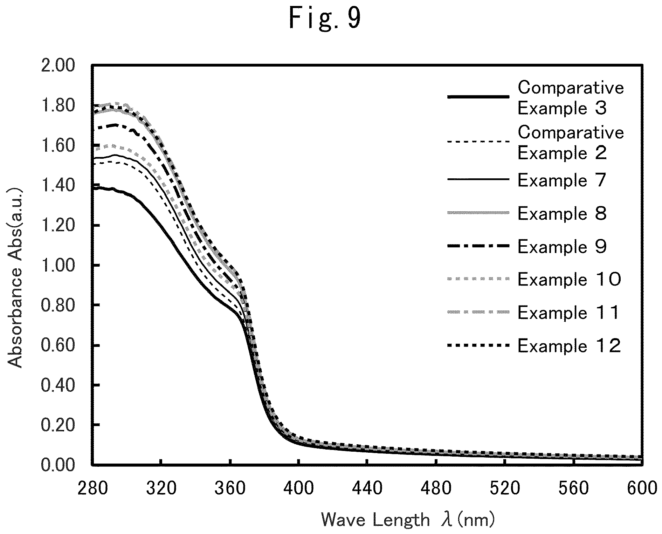

Next, based on the compositions shown in Table 3, the products of Examples 7 to 12 obtained as described above were dispersed, and the ultraviolet absorption ability of the obtained compositions was evaluated. The results are shown in FIG. 9.

Note that in Comparative Example 2, the raw material powder was added to a liquid medium as-is without being pulverized, and dispersed using an Agi-Homo Mixer, and the ultraviolet absorption ability thereof was evaluated. The results are shown in FIG. 9.

In Comparative Example 3, the ultraviolet absorption ability was evaluated in the same manner as Comparative Example 2, except that raw material powder was not added. The results are shown in FIG. 9.

Furthermore, the average particle diameter of the powder of each prepared composition was determined in the same manner as described above. The obtained particle diameter distribution is shown in Table 3.

[Table 3]

**(Table 3)**

| | | Example 7 | Example 8 | Example 9 | Example 10 | Example 11 | Example 12 | Comparative Example 2 | Comparative Example 3 |
|---|---|---|---|---|---|---|---|---|---|
| Products of Table 2 or compound having ultraviolet absorption ability | Form | Mixed powder 1 | Dispersion (slurry) 1 | Dispersion (slurry) 2 | Mixed powder 2 | Dispersion (slurry) 3 | Dispersion (slurry) 4 | Raw material powder | - |
| | Blending amount (mass%) | 5.0 | 10 | 10 | 10 | 20 | 20 | 5.0 | - |
| Liquid medium | Phenyl-modifie d nonvolatile silicone | 5.0 | 2.5 | 2.5 | 5.0 | - | - | 5.0 | 5.0 |
| | Nonvolatile dimethicone | 5.0 | 2.5 | 2.5 | 5.0 | - | - | 5.0 | 5.0 |
| Additional powder | Plate-like barium sulfate (extender pigment) | 5.0 | 5.0 | 5.0 | - | - | - | 5.0 | 5.0 |
| Additional components | Additional components | Balance | Balance | Balance | Balance | Balance | Balance | Balance | Balance |
| Total (% by mass) | | 100 | | | | | | | |
| Particle diameter distribution of powder (µm) | | 75 to 120 | 60 to 100 | 50 to 100 | 80 to 150 | 60 to 100 | 50 to 80 | 300 to 800 | - |

The "additional components" of Table 3 above are as follows:

| | |
|---|---|
| Water balance | |
| Ethanol | 10% by mass |
| PEG/PPG-9/2 dimethyl ether | 0.5% by mass |
| PEG/PPG-14/7 dimethyl ether | 0.5% by mass |
| Glycerin | 1.0% by mass |
| Disteardimonium hectorite | 0.5% by mass |
| Dextrin fatty acid ester | 1.0% by mass |
| PEG-9 polydimethylsiloxyethyl dimethicone | 2.0% by mass |
| Diisopropyl sebacate | 2.0% by mass |
| Nonvolatile dimethicone | 5.0% by mass |
| Volatile dimethicone | 12% by mass |
| Hydrophobized fine particle titanium oxide | 9.0% by mass |
| Hydrophobized fine particle zinc oxide | 18% by mass |
| Spherical silicone rubber powder | 3.0% by mass |
| Spherical cross-linked PMMA powder | 1.0% by mass |
| Spherical silica | 3.0% by mass |
| Chelating agent | appropriate amount |
| (Total: 100% by mass) | |

As is clear from the results of FIG. 9, as compared to Comparative Example 2, the ultraviolet absorbing powders of Examples 7 to 12 had significantly improved ultraviolet absorption ability in the UVA region (wavelength 320 to 400 nm) and UVB region (wavelength 290 to 320 nm).

### <<Examples 13 to 16 and Comparative Examples 4 and 5>>

The cosmetics (liquid cosmetics: oil-based) of Examples 13 to 16 and Comparative Examples 4 and 5 were produced based on the compositions shown in Table 4 below. The obtained cosmetics were evaluated for ultraviolet absorption ability and feeling upon use based on the following criteria.

Note that in Comparative Examples 4 and 5, the raw material powder was blended as-is without being pulverized. Furthermore, in Examples 13 and 15, the raw material powder was pulverized and blended as the ultraviolet absorbing powder. In Examples 14 and 16, the raw material powder and an extender pigment (hydrophobically treated barium sulfate) were pulverized together and then blended.

### <Ultraviolet Absorption Ability>

The UV absorption of the cosmetics of each Example and Comparative Example was measured, and the UV absorption improvement rate of Examples 13 and 14 was determined using the absorbance at 300 nm of Comparative Example 4 as a baseline (100%). Furthermore, the ultraviolet absorption improvement rates of Examples 15 and 16 were determined using Comparative Example 5 as a baseline (100%). The results are shown in Table 4.

### <Evaluation of Feeling Upon Use>

A comprehensive evaluation was conducted by three expert panelists to determine whether or not a coarse feeling could be experienced, the feeling upon use of the cosmetic of each Example and Comparative Example was evaluated based on the following criteria, and the obtained results are shown in Table 4.
- the case in which a smooth finish with no coarse feeling presumably due to the powder was experienced is evaluated as "A";
- the case in which a dry feeling upon use with no coarse feeling presumably due to the powder was experienced is evaluated as "B"; and
- the case in which a coarse feeling presumably due to the powder with no suitability for use was experienced is evaluated as "C."

[Table 4]

**(Table 4)**

| Composition | | Comparative Example 4 | Example 13 | Example 14 | Comparative Example 5 | Example 15 | Example 16 |
|---|---|---|---|---|---|---|---|
| Water | Water | Balance | Balance | Balance | Balance | Balance | Remainder |
| Alcohol | Ethanol | 10 | 10 | 10 | 10 | 10 | 10 |
| Moisturizer | PEG/PPG-9/2 dimethyl ether | 0.5 | 0.5 | 0.5 | 0.5 | 0.5 | 0.5 |
| | PEG/PPG-14/7 dimethyl ether | 0.5 | 0.5 | 0.5 | 0.5 | 0.5 | 0.5 |
| | Glycerin | 1.0 | 1.0 | 1.0 | 1.0 | 10 | 1.0 |
| Thickener | Disteardimonium hectorite | 0.5 | 0.5 | 0.5 | 0.5 | 0.5 | 0.5 |
| | Dextrin fatty acid ester | 1.0 | 1.0 | 1.0 | 1.0 | 1.0 | 1.0 |
| Activator | PEG-9 polydimethylsiloxyethyl dimethicone | 2.0 | 2.0 | 2.0 | 2.0 | 2.0 | 2.0 |
| Oil content | Diisopropyl sebacate | 2.0 | 2.0 | 2.0 | 2.0 | 2.0 | 2.0 |
| | Nonvolatile dimethicone | 10 | 10 | 10 | 10 | 10 | 10 |
| | Volatile dimethicone | 20 | 20 | 20 | 20 | 20 | 20 |
| | Isostearic acid | 1.0 | 1.0 | 1.0 | 1.0 | 1.0 | 1.0 |
| UV absorber | Octocrylene | - | - | - | 2.0 | 2.0 | 2.0 |
| | Bisethylhexyloxyphenolmethoxyp henyltriazine | - | - | - | 1.0 | 1.0 | 1.0 |
| | Diethylaminohydroxybenzoylhex yl benzoate | - | - | - | 1.0 | 1.0 | 1.0 |
| | Ethylhexyltriazone | - | - | - | 1.0 | 1.0 | 1.0 |
| | Ethylhexyl methoxycinnamate | - | - | - | 5.0 | 5.0 | 5.0 |
| | Compound 1 | 5.0 | 5.0 | 5.0 | 5.0 | 5.0 | 5.0 |
| | Compound 1 blending method | Normally added without pulverizi ng | Pulverized and b lended | Pulverized with extender pigment and blended | Normally added without pulverizi ng | Pulverized and b lended | Pulverized with extender pigment and blended |
| UV scattering agent | Hydrophobized fine particle tita nium oxide | 12 | 12 | 12 | 4.0 | 4.0 | 4.0 |
| | Hydrophobized fine particle zin c oxide | 20 | 20 | 20 | 10 | 10 | 10 |
| Usability pow der | Spherical silicone rubber powde r | 3.0 | 3.0 | 3.0 | 3.0 | 3.0 | 3.0 |
| | Hydrophobized barium sulfate p owder (extender pigment) | - | - | 5.0 | - | - | 5.0 |
| | Spherical cross-linked PMMA p owder | 1.0 | 1.0 | 1.0 | 1.0 | 1.0 | 1.0 |
| | Spherical silica | 3.0 | 3.0 | 3.0 | 3.0 | 3.0 | 3.0 |
| Chelating age nt | Chelating agent | Appropriate amt | Appropriate amt | Appropriate amt | Appropriate amt | Appropriate amt | Appropriate amt |
| Total (mass%) | | 100 | 100 | 100 | 100 | 100 | 100 |
| Evaluation | Absorbance improvement rate ( 300 nm) | 100% (baseline) | 125% | 130% | 100% (baseline) | 130% | 140% |
| | Feeling upon use | C | B | A | C | B | A |

### <<Examples 17 and 18 and Comparative Example 6>>

The cosmetics (liquid cosmetics: water-based) of Examples 17 and 18 and Comparative Example 6 were produced based on the compositions shown in Table 5 below. The obtained cosmetics were evaluated for ultraviolet absorption ability and feeling upon use in the same manner as described above.

Note that in Comparative Example 6, the raw material powder was blended as-is without being pulverized. Furthermore, in Example 17, the raw material powder was pulverized and blended as the ultraviolet absorbing powder. Furthermore, in Example 18, the raw material powder and an extender pigment (hydrophobized barium sulfate) were pulverized together and then blended.

Furthermore, the ultraviolet absorption improvement rates of Examples 17 and 18 were determined using the absorbance at 300 nm of Comparative Example 6 as a baseline (100%). The results are shown in Table 5.

[Table 5]

**(Table 5)**

| Composition | | Comparative Example 6 | Example 17 | Example 18 |
|---|---|---|---|---|
| Water | Water | Balance | Balance | Balance |
| Moisturizer | Glycerin | 4.0 | 4.0 | 4.0 |
| | 1,3-butylene glycol | 7.0 | 7.0 | 7.0 |
| | PEG/PPG-9/2 dimethyl ether | 5.0 | 5.0 | 5.0 |
| Thickener | Succinoglycan | 0.3 | 0.3 | 0.3 |
| | Sucrose fatty acid ester | 3.0 | 3.0 | 3.0 |
| | Carbomer | 0.1 | 0.1 | 0.1 |
| | (Acrylates/alkyl acrylate (C10-30)) cross polymer | 0.1 | 0.1 | 0.1 |
| | (Dimethylacrylamide/acryloyldimethyltaurine Na) crosspolymer | 0.3 | 0.3 | 0.3 |
| Surfactant | PEG-60 hydrogenated castor oil | 2.0 | 2.0 | 2.0 |
| Oil content | Diisopropyl sebacate | 2.0 | 2.0 | 2.0 |
| | Nonvolatile dimethicone | 5.0 | 5.0 | 5.0 |
| | Cyclomethicone | 12 | 12 | 12 |
| | Triethylhexanoin | 2.0 | 2.0 | 2.0 |
| | Isostearic acid | 1.0 | 1.0 | 1.0 |
| | Sorbitan sesquiisostearate | 0.5 | 0.5 | 0.5 |
| UV scattering agent | Dimethicone treated silica coated fine particle zinc oxide | 10 | 10 | 10 |
| UV absorber | Ethylhexyl methoxycinnamate | 5.0 | 5.0 | 5.0 |
| | Diethylaminohydroxybenzoylhexyl benzoate | 1.0 | 1.0 | 1.0 |
| | Bisethylhexyloxyphenolmethoxyphenyltriazine | 1.0 | 1.0 | 1.0 |
| | Compound 1 | 5.0 | 5.0 | 5.0 |
| | Compound 1 blending method | Normally added | Pulverized and blended | Pulverized with extender pigment and blended |
| Usability powder | Spherical urethane resin powder | 3.0 | 3.0 | 3.0 |
| | Hydrophobized barium sulfate powder (extender pigment) | - | - | 5.0 |
| Chelating agent | Citric acid | Appropriate amt | Appropriate amt | Appropriate amt |
| | Sodium citrate | Appropriate amt | Appropriate amt | Appropriate amt |
| | Chelating agent | Appropriate amt | Appropriate amt | Appropriate amt |
| | Preservative | Appropriate amt | Appropriate amt | Appropriate amt |
| | Addition method | Normally added without pulverizing | Pulverized and blended | Pulverized with powder and blended |
| Total (100% by mass) | | 100 | 100 | 100 |
| Evaluation | Absorbance improvement rate (300 nm) | 100% (baseline) | 110% | 120% |
| | Feeling upon use | C | B | A |

### <<Examples 19 and 20 and Comparative Example 7>>

The cosmetics (powder cosmetics) of Examples 19 and 20 and Comparative Example 7 were produced based on the compositions shown in Table 6 below. The obtained cosmetics were evaluated for ultraviolet absorption ability and feeling upon use in the same manner as described above.

Note that in Comparative Example 7, the raw material powder was blended as-is without being pulverized. Furthermore, in Example 19, the raw material powder was pulverized and blended as the ultraviolet absorbing powder. Furthermore, in Example 20, the raw material powder and hydrophobized barium sulfate, which is an extender pigment, were pulverized together and then blended.

Furthermore, the ultraviolet absorption improvement rates of Examples 19 and 20 were determined using the absorbance at 300 nm of Comparative Example 7 as a baseline (100%). The results are shown in Table 6.

[Table 6]

**(Table 6)**

| Composition Comparative Example 7 Example 19 Example 20 | | | | |
|---|---|---|---|---|
| UV scattering agent | Hydrophobized fine particle zinc oxide | 5.0 | 5.0 | 5.0 |
| | Hydrophobized fine particle titanium oxide | 5.0 | 5.0 | 5.0 |
| UV absorbing powder | Compound 1 | 5.0 | 5.0 | 5.0 |
| | Compound 1 blending method | Normally added without pulverizing | Pulverized and blended | Pulverized with extender pigment and blended |
| Usability powder | PMMA resin powder | 7.0 | 7.0 | 7.0 |
| | silica | 2.0 | 2.0 | 2.0 |
| | Mica (extender pigment) | 15 | 15 | 15 |
| | Synthetic phlogopite (extender pigment) | 8.0 | 8.0 | 8.0 |
| | Hydrophobized barium sulfate powder (extender pigment) | - | - | 5.0 |
| Oil content | Nonvolatile dimethicone | 5.0 | 5.0 | 5.0 |
| Oil content | Caprylyl methicone | 1.0 | 1.0 | 1.0 |
| UV absorbing oil | Ethylhexyl methoxycinnamate | 5.0 | 5.0 | 5.0 |
| Colorant | Hydrophobized pigment-grade titanium oxide | 10 | 10 | 10 |
| | Hydrophobized pigment grade zinc oxide | 2.0 | 2.0 | 2.0 |
| | Red iron oxide | 0.2 | 0.2 | 0.2 |
| | Yellow iron oxide | 1.5 | 1.5 | 1.5 |
| | Black iron oxide | 0.2 | 0.2 | 0.2 |
| | Mica titanium | 2.0 | 2.0 | 2.0 |
| Stabilizer | Citric acid | Appropriate amt | Appropriate amt | Appropriate amt |
| | Sodium citrate | Appropriate amt | Appropriate amt | Appropriate amt |
| | chelating agent | Appropriate amt | Appropriate amt | Appropriate amt |
| | Preservative | Appropriate amt | Appropriate amt | Appropriate amt |
| Total (100% by mass) | | 100 | 100 | 100 |
| Evaluation | Absorbance improvement rate (300 nm) | 100% (baseline) | 120% | 130% |
| | Feeling upon use | C | B | A |

## Claims

1. A cosmetic, comprising an ultraviolet absorbing powder,
wherein the ultraviolet absorbing powder is composed of particles of a compound I having a structure of formula (I) below: 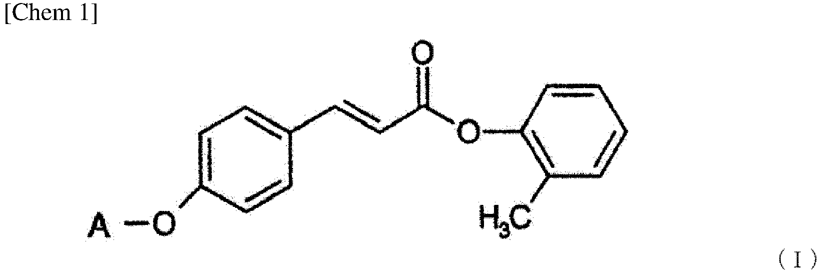
where -OA represents an alkoxy group, and
wherein in the ultraviolet absorbing powder, particles of the compound I having a longest diameter exceeding 500 µm are 1.0 or less per 100 particles of the compound I.

2. The cosmetic according to claim 1, wherein an average particle diameter of the ultraviolet absorbing powder is 350 µm or less.

3. The cosmetic according to claim 1 or 2, comprising a liquid medium,
wherein the ultraviolet absorbing powder is dispersed or settled in the liquid medium.

4. The cosmetic according to claim 3, wherein the liquid medium is nonvolatile, and a solubility of the ultraviolet absorbing powder in the liquid medium is 40 mg/ml or less.

5. The cosmetic according to claim 1 or 2, which is a powder cosmetic.

6. The cosmetic according to claim 5, further comprising an additional powder other than the ultraviolet absorbing powder.

7. The cosmetic according to claim 6, wherein the additional powder contains an extender pigment.

8. The cosmetic according to any one of claims 1 to 7, which is a sunscreen cosmetic.

9. An ultraviolet absorbing powder composed of particles of a compound I having a structure of formula (I) below: 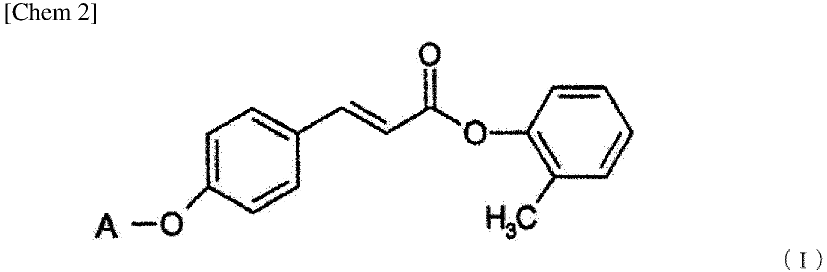
where -OA represents an alkoxy group, and
particles of the compound I having a longest diameter exceeding 500 µm are 1.0 or less per 100 particles of the compound I.

10. A dispersion, comprising a liquid medium, and
wherein the powder according to claim 9 is dispersed or settled in the liquid medium.

11. A mixed powder, comprising:
the powder according to claim 9, and
an additional powder other than the powder according to claim 9.

12. The powder according to claim 11, wherein the additional powder contains an extender pigment.

13. A production method for an ultraviolet absorbing powder, comprising pulverizing particles of a compound I having a structure of formula (I) below: 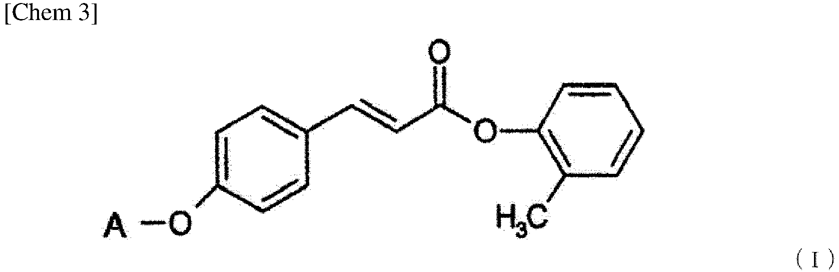 where -OA represents an alkoxy group.

14. The production method according to claim 13, wherein the pulverizing is such that particles of the compound I having a longest diameter exceeding 500 µm are 1.0 or less per 100 particles of the compound I.

15. The production method according to claim 13 or 14, wherein the pulverizing is performed using a wet pulverizer.

16. The production method according to claim 15, wherein a roller mill is used as the wet pulverizer.

17. The production method according to claim 15 or 16, wherein the pulverizing is performed after dispersing the particles of the compound I in a liquid medium.

18. The production method according to claim 13 or 14, wherein the pulverizing is performed using a dry pulverizer.

19. The production method according to claim 18, wherein the pulverizing is performed along with additional particles other than the particles of the compound I.

20. The production method according to claim 19, wherein the additional particles comprise an extender pigment.
